Europäisches Patentamt

European Patent Office

Office européen des brevets

(11) Numéro de publication : **0 368 696 B1**

(19)

(12) **FASCICULE DE BREVET EUROPEEN**

(45) Date de publication du fascicule du brevet :
**24.06.92 Bulletin 92/26**

(51) Int. Cl.⁵ : **C07C 59/52,** C07C 51/367

(21) Numéro de dépôt : **89402790.3**

(22) Date de dépôt : **10.10.89**

(54) **Nouveau procédé de fabrication industrielle du parahydroxymandélate de sodium.**

(30) Priorité : **08.11.88 FR 8814575**

(43) Date de publication de la demande :
**16.05.90 Bulletin 90/20**

(45) Mention de la délivrance du brevet :
**24.06.92 Bulletin 92/26**

(84) Etats contractants désignés :
**AT BE CH DE ES FR GB GR IT LI LU NL SE**

(56) Documents cités :
**EP-A- 0 024 181**
**FR-A- 2 427 322**

(56) Documents cités :
**HOUBEN-WEYL: METHODEN DER ORGANIS-
CHEN CHEMIE, 4 édition, 1976, tome VI/Ic, 2.
partie, page 1036, Georg Thieme VerlagStuttgart, RFA**
**PATENT ABSTRACTS OF JAPAN, vol. 4, no.
155 (C-29)(697), 29 octobre 1980; & JP-A-55 102
537**
**PATENT ABSTRACTS OF JAPAN, vol. 3, no. 83
(C-52), 28 juillet 1979; & JP-A-54 61 142**

(73) Titulaire : **SOCIETE FRANCAISE HOECHST
TOUR ROUSSEL HOECHST 1 Terrasse Bellini
F-92800 Puteaux (FR)**

(72) Inventeur : **Christidis, Yani
53 Boulevard de la Villette
F-75019 Paris (FR)**

(74) Mandataire : **Rinuy, Santarelli
14, avenue de la Grande Armée
F-75017 Paris (FR)**

## Description

La présente invention concerne un nouveau procédé de fabrication industrielle du parahydroxymandélate de sodium.

Le parahydroxymandélate de sodium cristallisé pur anhydre ou avec une molécule d'eau est décrit dans la littérature (brevets français 2.426.669, 2.427.322) et il permet d'accéder très aisément et avec d'excellents rendements, à l'acide parahydroxymandélique. Cet acide ainsi que ses sels d'ammonium ou de métal alcalin ou alcalino-terreux, sont des matières premières précieuses pour accéder notamment à divers intermédiaires de synthèse tels que le parahydroxybenzaldéhyde, l'acide parahydroxyphénylacétique qui connaissent des débouchés importants dans l'industrie pharmaceutique.

On sait préparer l'acide parahydroxymandélique ainsi que ses sels par de nombreuses méthodes, et plus particulièrement, par réaction de l'acide glyoxylique avec le phénol, à chaud, en milieu aqueux et en présence d'un hydroxyde de métal alcalin (brevets français N° 2.427.322, 2.440.350, 2.456.722, 2.495.137 et 2.426.669). Cette condensation n'est pas régiosélective et à coté de l' acide parahydroxymandélique, on forme des quantités relativement importantes d'acide orthohydroxymandélique et d'acide hydroxy-4 benzènediglycolique-1,3. De plus, cette condensation nécessite de travailler en milieu aqueux dilué, avec des excès importants de phénol et de soude qui entrainent de coûteux traitements d'isolement du produit cherché et de recyclage, ou éventuellement de destruction, des eaux-mères.

Afin d'obvier à tous ces inconvénients, la Demanderesse a découvert à présent qu'il est possible d'obtenir avantageusement le parahydroxymandélate de sodium cristallisé pur avec un bon rendement en s'affranchissant lors de la condensation de l'acide glyoxylique avec le phénol de la présence d'une base minérale forte, en faisant réagir de l'acide glyoxylique, en solution aqueuse, avec du phénol en excès, en présence uniquement d'une amine tertiaire, insoluble ou très peu soluble dans l'eau et liquide à la température ambiante, puis en salifiant l'acide parahydroxymandélique ainsi obtenu avec une solution aqueuse d'hydroxyde de sodium.

Selon le procédé de l'invention, la condensation de l'acide glyoxylique avec le phénol en excès, en présence d'une amine tertiaire liquide à la température ambiante et insoluble ou très peu soluble dans l'eau, est réalisée à une température comprise entre 15°C et 80°C, avantageusement entre 40°C et 60°C.

Comme amines tertiaires liquides à la température ambiante et insolubles ou peu solubles dans l'eau, on peut citer la tributylamine, la trioctylamine, la triisooctylamine ou leurs mélanges ainsi que des mélanges divers, commerciaux, d'amines tertiaires, tels que ceux commercialisés par la Demanderesse sous la dénomination HOSTAREX® dès lors qu'ils présentent les conditions requises citées précédemment. Dans le cas présent, peu soluble dans l'eau signifie qu'à 20°C la solubilité aqueuse est inférieure ou égale à celle de la tributylamine.

On utilise l'acide glyoxylique en solution aqueuse, avantageusement en solution aqueuse de concentrations supérieures à 50 % en poids et préférentiellement en solution aqueuse à 65 ± 2 % en poids.

Selon le procédé de l'invention, on utilise généralement un excès de 2 à 8 moles de phénol et de 1 à 5 moles d'amine tertiaire par mole d'acide glyoxylique mis en oeuvre, puis en fin de réaction, on introduit une solution aqueuse d'hydroxyde de sodium de manière à salifier l'acide parahydroxymandélique formé.

En fin de réaction, on isole le parahydroxymandélate de sodium cristallisé avec une molécule d'eau par des moyens connus en soi. Avantageusement, en fin de réaction, on introduit dans le milieu réactionnel une solution aqueuse d'hydroxyde de sodium puis après décantation du système biphasique obtenu, on isole de la phase aqueuse, éventuellement concentrée, le parahydroxymandélate de sodium cristallisé avec une molécule d'eau, sachant qu'il perd son eau de cristallisation par séchage à 110°C en fournissant le parahydroxymandélate de sodium cristallisé anhydre.

Les exemples suivants sont donnés à titre illustratif et nullement limitatif de l'invention.

### Exemple 1

On mélange à 20°C, sous agitation et en atmosphère inerte :
- 69,5 g (0,375 mole) de tributylamine,
- 188,2 g (2 moles) de phénol, puis, dans cette solution agitée, on introduit en 30 minutes:
- 28,48 g d'une solution aqueuse d'acide glyoxylique à 65 % en poids, soit 0,25 mole.

L'émulsion ainsi obtenue est ensuite chauffée à 40°C en 30 minutes, puis à 60°C en 30 minutes, et enfin on l'abandonne 60 minutes à cette température avant de la refroidir à 45°C. A cette température, on introduit 203 g d'une solution aqueuse d'hydroxyde de sodium à 8,87 % en poids, soit 0,45 mole, puis on décante. La phase organique (232,4 g) est conservée pour être recyclée et la phase aqueuse (253 g) est lavée trois fois avec 50 ml de diméthyl-1,1 méthoxy-1 propane, ce qui permet de récupérer 12,65 g (0,134 mole) de phénol. A ce stade, l'analyse d'une prise d'essai par chromatographie en phase liquide révèle que la phase aqueuse contient pondéralement, à l'état salifié, 15 % (35,3 g) d'acide parahydroxymandélique soit un rendement de 84 %, 2,1 % (4.9 g) d'acide orthohydroxymandélique et 0,1 % (0,23 g) d'acide hydroxy-4 benzènediglycolique-1,3 et qu'elle est exempte de phénol et de tributylamine. La phase aqueuse est ensuite concentrée sous vide, à chaud à 130 g environ, puis elle est abandonnée à la cristallisation vers 5°C et enfin elle est filtrée. Les cristaux recueillis sont lavés avec un mélange glacé eau-isopropanol 1-1 (v/v) puis séchés sous vide à 40°C à poids constant. On isole ainsi 38,5 g (0,185 mole) de parahydroxymandélate de sodium cristallisé avec une molécule d'eau soit un rendement de 74 % de la théorie calculée par rapport à l'acide glyoxylique mis en oeuvre.

La phase organique est recyclée directement dans une seconde opération identique après lui avoir ajouté le phénol consommé dans l'opération précédente, soit 35,5 g (0,377 mole).

On obtient ainsi de nouveau 38,4 g (0.185 mole) de parahydroxymandélate de sodium cristallisé avec une molécule d'eau.

Exemple 2

On mélange à 20°C, sous agitation et en atmosphère inerte :
– 132,6 g (0,375 mole) de triisooctylamine,
– 141,15 g (1.5 mole) de phénol, puis dans cette solution agitée, on introduit en 345 minutes, à 20 ± 3°C :
– 37 g d'une solution aqueuse d'acide glyoxylique à 50 % en poids, soit 0,25 mole.

L'introduction terminée, on introduit à la température ambiante, 200 g d'une solution aqueuse d'hydroxyde de sodium à 5 % en poids, soit 0,25 mole, puis on décante le milieu réactionnel biphasique obtenu. La phase organique (253,5 g) est conservée pour être recyclée et la phase aqueuse (251,6 g) est analysée par chromatographie en phase liquide.

Ces analyses indiquent la présence de 13,3 % (33,41 g, 0,199 mole) d'acide parahydroxymandèlique, 1,1% (2,76 g, 16,4 mmoles) d'acide orthohydroxymandélique, 2,3 % (5,78 g, 61 ,4 mmoles) de phénol et 0,2 % (0,5 g, 2 mmoles) d'acide hydroxy-4 benzènediglycolique-1 ,3.

A ce stade, le rendement est de 79,6 % de la théorie calculée par rapport à l'acide glyoxylique mis en oeuvre. La phase aqueuse est ensuite traitée comme dans l'exemple 1 et on isole ainsi 36,4 g (0, 175 mole) de parahydroxymandélate de sodium pur cristallisé avec une molécule d'eau, soit un rendement de 70 % de la théorie calculée par rapport à l'acide glyoxylique mis en oeuvre.

Revendications

1. Procédé de fabrication industrielle du parahydroxymandélate de sodium, caractérisé en ce que l'on fait réagir de l'acide glyoxylique en solution aqueuse avec un excès de phénol en présence d'une amine tertiaire insoluble ou peu soluble dans l'eau et liquide à la température ambiante, puis que l'on salifie l'acide parahydroxymandélique ainsi obtenu avec de l'hydroxyde de sodium en solution aqueuse.

2. Procédé selon la revendication 1, caractérisé en ce que la réaction de l'acide glyoxylique en solution aqueuse avec le phénol en excès est effectuée à une température comprise entre 15°C et 80°C.

3. Procédé selon la revendication 2, caractérisé en ce que cette réaction est effectuée à la température de 20 ± 3°C.

4. Procédé selon l'une quelconque des revendications 1 à 3, caractérisé en ce que l'amine tertiaire est la tributylamine.

5. Procédé selon l'une quelconque des revendications 1 à 3, caractérisé en ce que l'amine tertiaire est la triisooctylamine.

Claims

1. Process for the industrial manufacture of sodium parahydroxymandelate, characterized in that glyoxylic acid in aqueous solution is reacted with an excess of phenol in the presence of a tertiary amine, insoluble or slightly soluble in water and liquid at ambient temperature, then the parahydroxymandelic acid thus obtained is salified with sodium hydroxide in aqueous solution.

2. Process according to claim 1, characterized in that the reaction of the glyoxylic acid in aqueous solution with an excess of phenol is carried out at a temperature between 15°C and 80°C.

3. Process according to claim 2, characterized in that this reaction is carried out at a temperature of 20 ± 3°C.

4. Process according to any one of claims 1 to 3, characterized in that the tertiary amine is tributylamine.

5. Process according to any one of claims 1 to 3, characterized in that the tertiary amine is triisooctylamine.

Patentansprüche

1. Verfahren zur industriellen Herstellung von Natrium-p-hydroxymandelat, dadurch gekennzeichnet, daß Glyoxylsäure in wässeriger Lösung mit einem Überschuß von Phenol in Anwesenheit eines in Wasser unlöslichen oder wenig löslichen und bei Umgebungstemperatur flüssigen tertiären Amins umgesetzt wird, worauf von der so erhaltenen p-Hydroxymandelsäure mit Natriumhydroxid in wässeriger Lösung ein Salz gebildet wird.

2. Verfahren nach Anspruch 1, dadurch gekennzeichnet, daß die Reaktion der Glyoxylsäure in wässeriger Lösung mit dem überschüssigen Phenol bei einer Temperatur von 15 bis 80°C durchgeführt wird.

3. Verfahren nach Anspruch 2, dadurch gekennzeichnet, daß diese Reaktion bei einer Temperatur von 20 ± 3°C durchgeführt wird.

4. Verfahren nach einem der Ansprüche 1 bis 3, dadurch gekennzeichnet, daß das tertiäre Amin Tributylamin ist.

5. Verfahren nach einem der Ansprüche 1 bis 3, dadurch gekennzeichnet, daß das tertiäre Amin Triisooctylamin ist.